## Europäisches Patentamt

## European Patent Office

## Office européen des brevets

(19)

(11) Publication number:

# 0 081 156
# B1

(12) # EUROPEAN PATENT SPECIFICATION

(45) Date of publication of patent specification: **13.07.88**

(51) Int. Cl.⁴: **C 12 M 1/00, C 12 M 3/00 //**
**C12N1/12, C12N1/20**

(21) Application number: **82110916.2**

(22) Date of filing: **25.11.82**

(54) **Apparatus for photosynthesis.**

(30) Priority: **03.12.81 JP 194921/81**
**10.12.81 JP 199873/81**
**10.12.81 JP 199874/81**
**10.12.81 JP 199875/81**

(43) Date of publication of application:
**15.06.83 Bulletin 83/24**

(45) Publication of the grant of the patent:
**13.07.88 Bulletin 88/28**

(84) Designated Contracting States:
**CH DE FR GB IT LI SE**

(56) References cited:
**WO-A-79/00282**
**DE-A-2 039 614**
**FR-A-1 416 096**
**US-A-3 218 758**
**US-A-3 986 297**

(73) Proprietor: **Mori, Kei**
**3-16-3-501, Kaminoge**
**Setagaya-ku Tokyo (JP)**

(72) Inventor: **Mori, Kei**
**3-16-3-501, Kaminoge**
**Setagaya-ku Tokyo (JP)**

(74) Representative: **Patentanwälte Grünecker, Dr.**
**Kinkeldey, Dr. Stockmair, Dr. Schumann, Jakob,**
**Dr. Bezold, Meister, Hilgers, Dr. Meyer-Plath**
**Maximilianstrasse 58**
**D-8000 München 22 (DE)**

Courier Press, Leamington Spa, England.

**Description**

The present invention relates to an apparatus for photosynthesis comprising of a photosynthetic reaction bath and a plurality of photoradiators arranged in said reaction bath having a narrow tubular configuration.

Photosynthetic apparatus heretofore proposed include an apparatus for culturing chlorella (unicellular microorganism containing chlorophyl). Difficulty experienced in culturing chlorella is that illumination with intensities higher than a certain level destroys the chlorophyl and produces a toxin (phaeophorbite) while illumination with intensities lower than a certain level fails to cause photosynthesis altogether. It is therefore a primary requisite for effective photosynthesis that all the cells containing photosynthetic substances be supplied with constant light of an even distribution. Generally, organisms in a swarm increase not only their multiplying ability per unit volume but their resistance to other fungi. The culturing efficiency, therefore is poor unless a predetermined light radiating area per one liter of culture medium is ensured. With this in view, it has been customary to promote the transmission of light by reducing the number of photosynthetic substances (individuals). This involves an inconsistency, however, because an increase in the number of individuals lowers the transmissibility to light and, therefore, requires collection of the individuals, but the resulting decrease in the number of individuals weakens the resistance to the funge. Another drawback hitherto encountered is that the light intensity is excessively high for the individuals near a light source but is insufficient for those remote from the light source and, additionally, the light is absorbed by water causing its wavelength component to be varied. An ideal situation is, therefore, that photosynthetic substances be passed through a very narrow clearance while a predetermined intensity of light is directed perpendicular to the clearance. Then, sufficient light will be evenly applied to all the cells containing photosynthetic substances with a minimum of attenuation and without any change in its wavelength component. A photosynthetic apparatus present in use includes a number of fluorescent lamps arranged in a reaction bath (e.g. chlorella culturing bath) and causes photosynthetic substances to flow through the gaps between the lamps. However, the use of fluorescent lamps renders the apparatus bulky, increases power consumption and requires an awkward measure against heat generation by the lamps. Moreover, fluorescent lamps generally have peaks at specific wavelengths and those having a peak in their green component, which is harmful for chlorophyl, are unsuitable for photosynthesis. Apparently, sunlight or like natural light is most adequate for culturing chlorella and the like.

In culturing chlorella, for example, one liter of culture medium extends over an area of 1 $m^2$ when spread to a thickness of 1 mm. Meanwhile, when the culture medium is laid on a 1 $m^2$ plane light source to a thickness of 1 mm, the intensity of the light source being assumed to be 1000 lx, the intensity after the light has passed through the 1 mm thick culture medium is reduced to about 30 lx in the case of a high density chlorella culture medium for enhancing the multiplying ability. Thus, hardly any light is allowed to reach chlorella located opposite to the plane light source. Although such a problem may be solved if plane light sources are located at both sides of the 1 mm thick culture medium, such results in generation of a significant amount of heat when the light sources comprise fluorescent lamps, as has been the case with conventional apparatuses.

From the W-O-79/00282 document an apparatus for photosynthesis is known, which utilizes photoradiators in the form of light guides formed by a bundle of optical fibers. The light guides disperse solar light in the reaction bath.

However, there is no disclosure in this document regarding a proper arrangement of said light guides.

It is the object of the present invention to provide an apparatus for photosynthesis which effectively promotes photosynthesis and which can be readily scaled-up as required.

To accomplish this object the inventive apparatus is characterized therein that the outer surfaces of said photoradiators are spaced apart by a minimum distance, which is greater than zero and not more than about 2 mm, in a way that the contact area per culture medium ($S/W_1$; $S'/W_2$; $S''/W_3$; wherein S, S' and S'' is the area, in square metres, of the photoradiators in contact with the culture medium and $W_1$, $W_2$ and $W_3$ is the volume of culture medium, in litres, allowed to flow through the space between the photoradiators) is greater than or equal to 1.

Preferred embodiments of the invention are claimed in the depending claims.

Brief description of the drawings

Figure 1 is a fragmentary enlarged cross-section of a photosynthetic apparatus embodying the present invention;

Figure 2 is a view of a photoradiator applicable to the apparatus shown in Figure 1;

Figure 3 is a fragmentary enlarged cross-section of another embodiment of the present invention;

Figure 4 is a view of a photoradiator applicable to the apparatus shown in Figure 3;

Figure 5 is a section along line VV of Figure 4;

Figure 6 is a fragmentary enlarged cross-section of another embodiment of the present invention;

Figure 7 is a view of a photoradiator applicable to the apparatus shown in Figure 6;

Figure 8 is a section along line VIII—VIII of Figure 7;

Figure 9 is a fragmentary enlarged cross-section of still another embodiment of the present invention;

2

Figure 10 is a view of a photoradiator applicable to the apparatus shown in Figure 9; and Figure 11 is a section along line XI—XI of Figure 10.

Description of the preferred embodiments

While the apparatus for photosynthesis of the present invention is susceptible of numerous physical embodiments, depending upon the environment and requirements of use, substantial numbers of the herein shown and described embodiments have been made, tested and used, and all have performed in an eminently satisfactory manner.

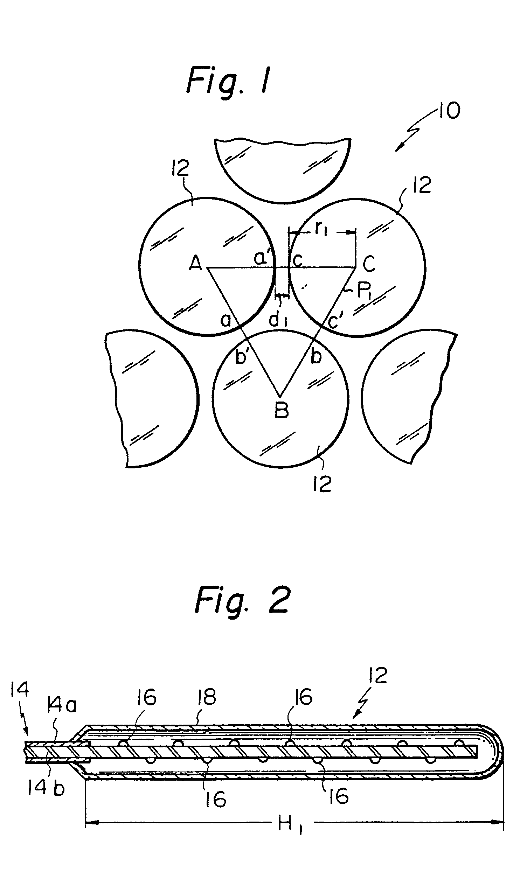

Referring to Figure 1 of the drawings, a photosynthetic reaction bath in accordance with the present invention is shown and generally designated by the reference numeral 10. A number of photoradiators 12 are disposed in the reaction bath 10 at regular spacings $d_1$. Each photoradiator 12 has a radius of $r_1$ cm.

Suppose that arbitrarily selected adjacent three of the photoradiators 12 have centers A, B and C as illustrated. Then, the triangle $P_1$ (ABC) has an area $S_1$ which is expressed as:

$$S_1 = (2r_1 + d_1)^2 \cos 30° \times \frac{1}{2} \qquad \text{Eq. (1)}$$

The sectors $Aaa' + Bbb' + Ccc'$ have an area $S_2$ which is given by:

$$S_2 = (\pi r_1^2 \times \frac{1}{6}) \times 3 = \pi r_1^2 \times \frac{1}{2} \qquad \text{Eq. (2)}$$

The area $S_3$ defined by $ab'bc'ca'$ is obtained as:

$$S_3 = S_1 - S_2 = \frac{1}{2}\{(2r_1 + d_1)^2 \cos 30° - \pi r_1^2\} \qquad \text{Eq. (3)}$$

Further, the arcs $aa' + bb' + cc'$ have a total length g which is:

$$g = 2\pi r_1 \times \frac{3}{6} = \pi r_1 \qquad \text{Eq. (4)}$$

Assuming that each photoradiator 12 has a length $H_1$ (see Figure 2), then the volume of culture medium $W_1$ allowed to flow through the area $S_3$ is expressed as:

$$W_1 = H_1 \times S_3 = \frac{H_1}{2}\{(2r_1 + d_1)^2 \cos 30° - \pi r_1^2\} \qquad \text{Eq. (5)}$$

The above-mentioned amount of culture medium $W_1$ contacts the photoradiators 12 over an area S which is:

$$S = H_1 \times g = H_1 \pi r_1 \qquad \text{Eq. (6)}$$

Hence, the contact area per unit culture medium, $S/W_1$, is given by:

$$S/W_1 = \frac{2\pi r_1}{(2r_1 + d_1)^2 \cos 30° - \pi r_1^2}$$

$$= \frac{H_1 \pi r_1}{\frac{H_1}{2}\{(2r_1 + d_1)^2 \cos 30° - \pi r_1^2\}} \qquad \text{Eq. (7)}$$

With respect to various values of the radius $r_1$ of the photoradiators 12 and distance $d_1$ between the adjacent photoradiators 12, the factors S, $W_1$ and $S/W_1$ were obtained as indicated in Table 1 below.

TABLE 1

| cm $d_1$ | cm $r_1$ | cm $H_1$ | cm$^2$ S | cm$^3$ $W_1$ | m$^2$/l $S/W_1$ |
|---|---|---|---|---|---|
| 0 | 0.5 | 30 | 47.12 | 1.209 | *3.896 |
| | 1.0 | 30 | 94.25 | 4.838 | *1.948 |
| | 2.0 | 30 | 188.50 | 19.351 | 0.974 |
| 0.05 | 0.5 | 30 | 47.12 | 2.541 | *1.855 |
| | 1.0 | 30 | 94.25 | 7.468 | *1.262 |
| | 2.0 | 30 | 188.50 | 24.58 | 0.767 |
| 0.1 | 0.5 | 30 | 47.12 | 3.937 | #1.1969 |
| | 1.0 | 30 | 94.25 | 10.16 | 0.9273 |
| | 2.0 | 30 | 188.50 | 29.87 | 0.6310 |
| 0.2 | 0.5 | 30 | 47.12 | 6.925 | 0.6805 |
| | 1.0 | 30 | 94.25 | 15.75 | 0.5984 |
| | 2.0 | 30 | 188.50 | 40.65 | 0.464 |
| 0.3 | 0.5 | 30 | 47.12 | 10.17 | 0.4633 |
| | 1.0 | 30 | 94.25 | 21.60 | 0.4364 |
| | 2.0 | 30 | 188.50 | 51.70 | 0.3646 |

It will be seen from Table 1 that the condition $S/W_1 \geqq 1$ previously discussed is satisfied by five different ratios $S/W_1$ as indicated by marks "*" and "#". However, the spacing $d_1$ which is zero is impractical because it allows no culture medium to be recirculated, while 0.5 mm of spacing $d_1$ is difficult to be accurately defined during manufacture. Thus, a ratio $S/W_1$ which can be put to practical use is the one marked "#" in Table 1 provided by the spacing $d_1$ of 1 mm and the diameter of 10 mm. Concerning the spacing $d_1$, it will be noted that about 1.2 mm at the maximum is allowable in view of the fact that bubbling is usually caused by carbon dioxide in the culture medium. To promote continuous operation, the reaction bath 10 may be provided with a hermetically sealed structure which avoids contamination to the culture medium, evaporation of water, etc. The number of photoradiators 12 can readily be increased while satisfying the condition $S/W_1 \geqq 1$, promoting the ease of scale-up of the apparatus. For the triangular photoradiators 12, the reaction bath may have a triangular, square, hexagonal or circular cross-section.

Referring to Figure 2, a preferred example of the photoradiators 12 applicable to the apparatus shown in Figure 1 is illustrated. The photoradiator 12 includes a light transmitting cable generally designated by the reference numeral 14. The cable 14 comprises a clad layer 14a, a core 14b, and a number of light diffusing members 16 each adhered to the core 14b. The assembly is hermetically encapsulated in a transparent casing 18. The solar rays are collected by an apparatus, not shown, and conducted through the cable 14. The light rays reached the interior of the casing 18 are diffused by the diffusers 16 to the outside (into the reaction bath 10) via the transparent casing 18. In the illustrated embodiment, such photoradiators 12 are arranged regularly in the reaction bath 10 so as to achieve the operation and effect previously discussed.

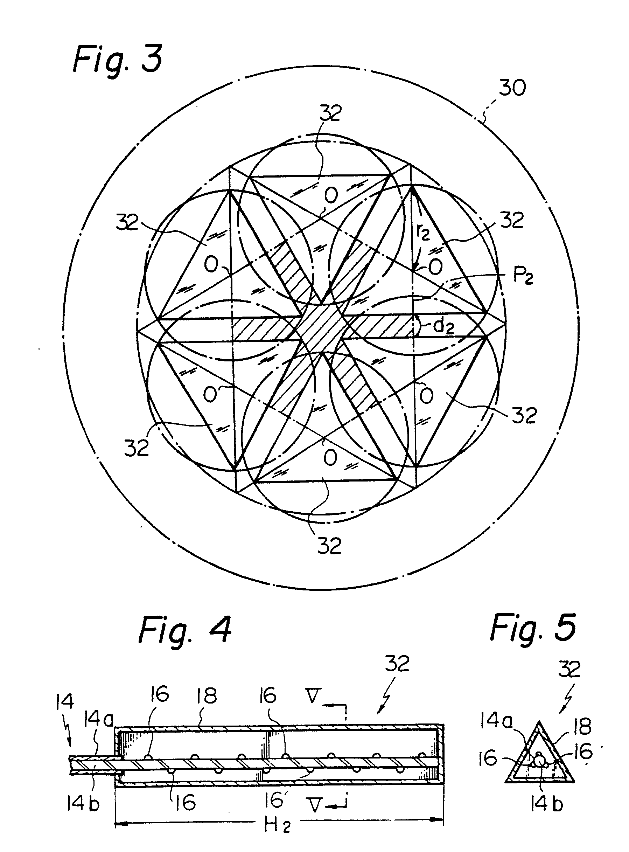

Referring to Figure 3, another embodiment of the photosynthetic apparatus of the present invention is shown. The apparatus comprises a photosynthetic reaction bath 30 in which a number of photoradiators 32 are arranged in neat order at a common spacing $d_2$. Each photoradiator 32 has an equilateral triangular cross-section and a radius of $r_2$ cm (one side being dimensioned $r_2 \times \cos 30°$).

As in the first embodiment, arbitrarly selected adjacent six of the photoradiators 32 are assumed to have centers O which define a hexagon $P_2$. Then, the total area (hatched in Figure 3) $S_4$ of the spacings or gaps within the hexagon $P_2$ is expressed as:

$$S_4 = 6\, d_2 r_2 \cos 30° + 3 d_2{}^2 \cos 30°$$

$$= 3\, d_2 \cos 30° \, (2r_2 + d_2) \qquad \text{Eq. (8)}$$

Supposing that the length $H_2$ of each photoradiator 32 is 30 cm, the volume $W_2$ of the culture medium allowed into the total gap is given by:

$$W_2 = H_2 \times S_4$$

$$= 30 \times 3\, d_2 \cos 30° \, (2r_2 + d_2)$$

$$= 90 \times d_2 \cos 30° \, (2r_2 + d_2) \qquad \text{Eq. (9)}$$

4

Meanwhile, the photoradiators 32 contact the culture medium over an area S which is:

$$S'=H_2 \times 12 \times r_2 \cos 30° = 360\ r_2 \cos 30° \qquad \text{Eq. (10)}$$

The various values indicated in Table 2 were obtained with respect to parameters which were the radius $r_2$ of each photoradiator 32 and the distance $d_2$ between the adjacent photoradiators 32.

TABLE 2

| cm $d_2$ | cm $r_2$ | cm $H_2$ | cm$^2$ $S'$ | cm$^3$ $W_2$ | m$^2$/l $S'/W_2$ |
|---|---|---|---|---|---|
| 0.05 | 0.5 | 30 | 155.88 | 4.092 | 3.809 |
|  | 1.0 | 30 | 311.77 | 7.989 | 3.902 |
|  | 2.0 | 30 | 623.54 | 15.78 | 3.951 |
| 0.1 | 0.5 | 30 | 155.88 | 8.574 | 1.818 |
|  | 1.0 | 30 | 311.77 | 16.37 | 1.905 |
|  | 2.0 | 30 | 623.54 | 31.96 | 1.951 |
| 0.17 | 0.5 | 30 | 155.88 | 15.50 | 1.005 |
|  | 1.0 | 30 | 311.77 | 28.75 | 1.084 |
|  | 2.0 | 30 | 623.54 | 55.25 | 1.128 |
| 0.18 | 0.5 | 30 | 155.88 | 16.55 | 0.942 |
|  | 1.0 | 30 | 311.77 | 30.58 | 1.019 |
|  | 2.0 | 30 | 623.54 | 56.64 | 1.013 |
| 0.2 | 0.5 | 30 | 155.88 | 18.71 | 0.833 |
|  | 1.0 | 30 | 311.77 | 34.29 | 0.909 |
|  | 2.0 | 30 | 623.54 | 65.47 | 0.952 |
| 0.3 | 0.5 | 30 | 155.88 | 30.39 | 0.513 |
|  | 1.0 | 30 | 311.77 | 53.78 | 0.579 |
|  | 2.0 | 30 | 623.54 | 100.54 | 0.620 |

As shown in Table 2, the ratio $S'/W_2$ satisfies the condition $S'/W_2 \geqq 1$ as long as the distance $d_2$ between the adjacent photoradiators 32 is not more than about 0.18 cm. It will be seen from Table 2 that the ratio $S'/W_2$ substantially depends upon the distance $d_2$ and is hardly effected by the diameter ($r_2$) of the photoradiators. Again, the culture medium is usually caused to bubble by carbon dioxide and this permits the distance $d_2$ to become somewhat larger than the upper limit mentioned, i.e. up to about 2 mm in practice. Thus, in this embodiment, photosynthesis can effectively occur without being effected by the size of the photoradiators 32 only if the gap $d_2$ between the photoradiators 32 is maintained not more than about 2 mm. It is possible, therefore, to use photoradiators of various sizes in combination. For example, some of the photoradiators 32 may comprise fluorescent lamps to emit artificial light while the rest may comprise photoradiators of a relatively small diameter to emit natural light from optical fibers. With this combination, photosynthesis will occur during daytime taking advantage of the sunlight and, during nighttime or the like, aided by the fluorescent lamps. Furthermore, because the photoradiators using optical fibers are capable of guiding sunlight (natural light) or artificial light emitted from a fluorescent lamp or xenon lamp as desired, various combinations of sunlight and artificial light are avalaible such as introducing sunlight during daytime and artificial light during nighttime or introducing sunlight into some of the photoradiators and artificial light into the rest. Due to the triangular cross-section, the photoradiators 32 can be arranged effectively in the reaction bath 30 if the reaction bath is provided with a triangular, square or hexagonal contour. Again, the reaction bath 30 can operate continuously without contamination to the culture medium or evaporation of water if provided with a hermetically sealed structure. The number of the photoradiators 32 can be readily increased under the condition concerned, i.e., $S'3/W_2 \geqq 1$, facilitating the scale-up of the apparatus.

Figures 4 and 5 illustrate an example of the photoradiators 32 which is suitable for emitting natural light for photosynthesis. In these drawings, the same elements as those shown in Figure 2 are designated by like reference numerals.

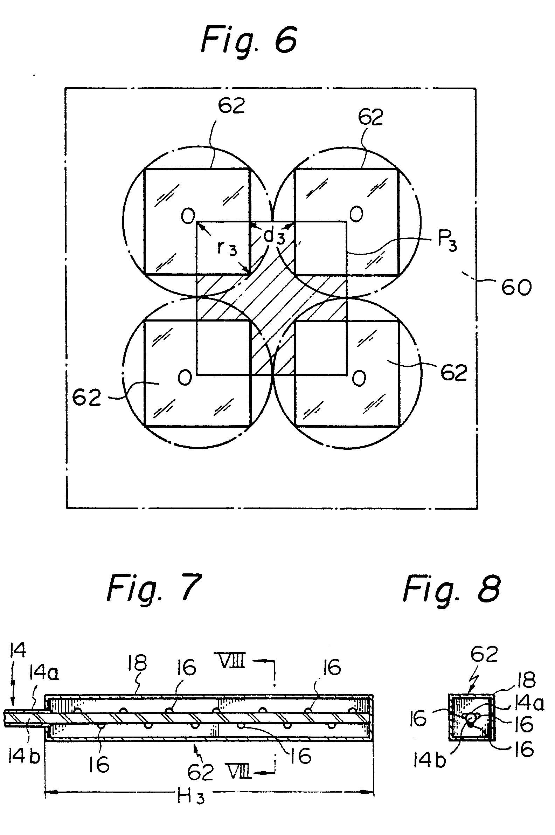

Referring to Figure 6, another embodiment of the present invention is shown which comprises a photosynthetic reaction bath 60. A number of photoradiators 62 are arranged in the reaction bath 60 at regular spacings of $d_3$. Each photoradiator 62 has a square cross-section with a radius of $r_3$ cm (one side being dimensioned $r_3 \times \cos 45°$).

0 081 156

Let it be supposed that arbitrarily selected four adjacent photoradiators 62 have centers O which define a square $P_3$. Then, the total area (hatched in Figure 6) $S_5$ of the gaps between the photoradiators 62 within the square $P_3$ is expressed as:

$$S_5 = r_3 \cos 45° \times d_3 \times 4 + d_3^2$$

$$= d_3(d_3 + 4r_3 \cos 45°) \qquad \text{Eq. (11)}$$

Where the length $H_3$ of the photoradiators 62 is 30 cm for example, the volume $W_3$ of the culture medium which the gaps can accommodate is given by:

$$W_3 = H_2 \times S_5$$

$$= H_3 d_3 (d_3 + 4r_3 \cos 45°) \qquad \text{Eq. (12)}$$

Meanwhile, the area $S''$ over which the light emitting surfaces of the photoradiators 62 contact the culture medium is obtained as:

$$S'' = 2r_3 \cos 45° \times 4 \times H_3$$

$$= (8r_3 \cos 45°) \times H_3 \qquad \text{Eq. (13)}$$

The factors $S''$, $W_3$ and $S''/W_3$ obtained with respect to various values of the radius $r_3$ of the photoradiators 62 and their gaps $d_3$ are indicated in Table 3.

TABLE 3

| cm $d_3$ | cm $r_3$ | cm $H_3$ | cm² $S''$ | cm³ $W_3$ | m²/l $S''/W_3$ |
|---|---|---|---|---|---|
| 0.05 | 0.5 | 30 | 84.85 | 2.1961 | 3.861 |
| | 1.0 | 30 | 169.7 | 4.3176 | 3.930 |
| | 2.0 | 30 | 339.41 | 8.563 | 3.965 |
| | 3.0 | 30 | 509.1 | 12.803 | 3.976 |
| 0.1 | 0.5 | 30 | 84.85 | 4.543 | 1.868 |
| | 1.0 | 30 | 169.7 | 8.785 | 1.932 |
| | 2.0 | 30 | 339.41 | 17.27 | 1.965 |
| | 3.0 | 30 | 509.1 | 25.76 | 1.977 |
| 0.17 | 0.5 | 30 | 84.85 | 8.078 | 1.050 |
| | 1.0 | 30 | 169.7 | 15.289 | 1.109 |
| | 2.0 | 30 | 339.41 | 29.712 | 1.142 |
| | 3.0 | 30 | 509.1 | 44.135 | 1.153 |
| 0.18 | 0.5 | 30 | 84.85 | 8.607 | 0.986 |
| | 1.0 | 30 | 169.7 | 16.243 | 1.045 |
| | 2.0 | 30 | 339.41 | 31.51 | 1.077 |
| | 3.0 | 30 | 509.1 | 46.78 | 1.088 |
| 0.2 | 0.5 | 30 | 84.85 | 9.685 | 0.8761 |
| | 1.0 | 30 | 169.7 | 18.171 | 0.9339 |
| | 2.0 | 30 | 339.41 | 35.141 | 0.9658 |
| | 3.0 | 30 | 509.1 | 52.112 | 0.9769 |
| 0.3 | 0.5 | 30 | 84.85 | 15.43 | 0.5499 |
| | 1.0 | 30 | 169.7 | 28.16 | 0.6027 |
| | 2.0 | 30 | 339.41 | 53.61 | 0.633 |

As shown in Table 3, the ratio $S''/W_3$ satisfies the condition $S''/W_3 \geq 1$ as long as the distance $d_3$ between the adjacent photoradiators 62 is not more than about 0.18 cm. It will be seen from Table 2 that the ratio $S''/W_3$ substantially depends upon the distance $d_3$ and is hardly effected by the diameter ($r_3$) of the photoradiators 62. Again, the culture medium is usually caused to bubble by carbon dioxide and this permits the distance $d_2$ to become somewhat larger than the upper limit mentioned, i.e. up to about 2 mm in practice. For the square photoradiators 62, the reaction bath may have a square or circular cross-section.

6

Figures 7 and 8 illustrate an example of the photoradiators 62 which is suitable for guiding and emitting natural light in connection with the embodiment shown in Figure 6. In these drawings, the same elements as those shown in Figure 2 are designated by the same reference numerals.

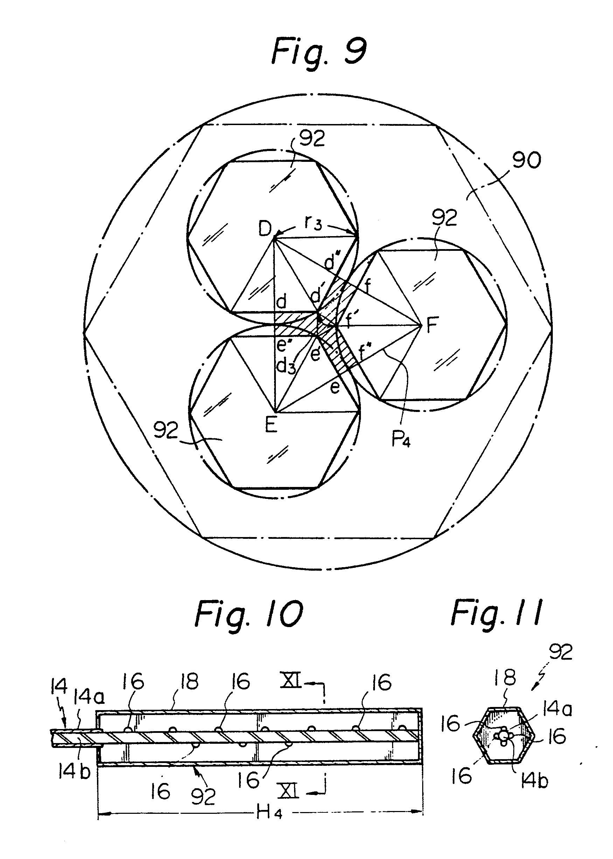

Referring to Figure 9, still another embodiment of the present invention is shown which comprises a photosynthetic reaction bath 90. Arranged regularly inside the reaction bath 90 are a number of photoradiators 92. Each of these photoradiators 92 has a hexagonal cross-section one side (radius) of which is $r_3$ cm long and is spaced $d_3$ from the adjacent photoradiators 92.

As in the foregoing embodiments, arbitrarily selected three of the photoradiators 92 have centers D, E and F, respectively. Lines DE, EF and FA interconnecting the centers D, E and F are assumed to intersect the light radiating surfaces of the photoradiators 92 at points d, e'', e, f'', f and d''. Further, the segments bisecting the interior angles of the triangle $P_4$ (DEF) are assumed to intersect the light emitting surfaces of the photoradiators 92 at points d', e' and f'. Then, the area $S_6$ of the triangle d'f'e' is expressed as:

$$S_6 = d_4{}^2 \cos 30° \times \tfrac{1}{2} \qquad\qquad \text{Eq. (14)}$$

The area $S_7$ of the tetragons dd'e'e''+ee'f'f''+ff'd'd'' is given by:

$$S_7 = \tfrac{1}{2} r_4 d_4 \times 3 \qquad\qquad \text{Eq. (15)}$$

The area $S_8$ of the gaps between the photoradiators 92 within the triangle $P_4$ is obtained as:

$$S_8 = S_6 + S_7 = \tfrac{1}{2} (d_4{}^2 \cos 30° + 3r_4 d_4) \qquad\qquad \text{Eq. (16)}$$

Supposing that each photoradiator 92 is $H_4$ long, the volume $W_4$ of the culture medium filling the gaps between the photoradiators 92 is expressed as:

$$W_4 = S_8 H_4 = \tfrac{1}{2} (d_4{}^2 \cos 30° + 3r_4 d_4) H_4 \qquad\qquad \text{Eq. (17)}$$

The area $S'''$ over which the light emitting surfaces of the photoradiators 92 contact the culture medium is provided as:

$$S''' = 3 r_4 H_4 \qquad\qquad \text{Eq. (18)}$$

The values $S'''$, $W_4$ and $S'''/W_4$ obtained with respect to various values of the distance $d_4$ between the photoradiators 92 and length of one side $r_4$ (radius) of each photoradiator are shown in Table 4.

TABLE 4

| cm $d_4$ | cm $r_4$ | cm $H_4$ | cm$^2$ $S'''$ | cm$^3$ $W_4$ | m$^2$/l $S'''/W_4$ |
|---|---|---|---|---|---|
| 0.05 | 0.5 | 30 | 45 | 1.157 | 3.889 |
|  | 1.0 | 30 | 90 | 2.282 | 3.944 |
|  | 2.0 | 30 | 180 | 4.532 | 3.972 |
|  | 3.0 | 30 | 270 | 6.782 | 3.981 |
| 0.1 | 0.5 | 30 | 45 | 2.380 | 1.891 |
|  | 1.0 | 30 | 90 | 4.630 | 1.944 |
|  | 2.0 | 30 | 180 | 9.130 | 1.972 |
|  | 3.0 | 30 | 270 | 13.630 | 1.981 |
| 0.15 | 0.5 | 30 | 45 | 3.667 | 1.227 |
|  | 1.0 | 30 | 90 | 7.042 | 1.278 |
|  | 2.0 | 30 | 180 | 13.793 | 1.305 |
|  | 3.0 | 30 | 270 | 20.532 | 1.315 |
| 0.18 | 0.5 | 30 | 45 | 4.473 | 1.006 |
|  | 1.0 | 30 | 90 | 8.520 | 1.050 |
|  | 2.0 | 30 | 180 | 16.62 | 1.083 |
|  | 3.0 | 30 | 270 | 24.72 | 1.092 |
| 0.2 | 0.5 | 30 | 45 | 5.020 | 0.896 |
|  | 1.0 | 30 | 90 | 9.520 | 0.945 |
|  | 2.0 | 30 | 180 | 18.520 | 0.972 |
|  | 3.0 | 30 | 270 | 27.520 | 0.981 |

It will be seen from Table 4 that the ratio $S'''/W_4$ satisfies the condition $S'''/W_4 \geq 1$ as long as the distance $d_4$ between the addjacent photoradiators 92 is not more than 0.18 cm, and that the ratio $S'''/W_4$ is substantially dependent upon the distance $d_4$ and hardly effected by the radius $(r_3)$ of the photoradiators 92. For the hexagonal photoradiators 92, the reaction bath may have a hexagonal or circular cross-section.

Figures 11 and 12 illustrate an example of the photoradiators 92 suitable for emitting natural light in connection with the embodiment shown in Figure 9. In these drawings, the same elements as those shown in Figure 2 are designated by like reference numerals.

In summary, it will be seen that the present invention provides a photosynthetic apparatus which can be effectively operated and readily scaled up due to the effective arrangement of photoradiators. Furthermore, the apparatus of the invention does not use any light source which contains a heat source such as a fluorescent lamp, thereby precluding the need for a measure against heat generation.

**Claims**

1. An apparatus for photosynthesis comprising a photo-synthetic reaction bath (10, 30, 60) and a plurality of photoradiators (12, 32, 62) arranged in said reaction bath, having a narrow tubular configuration, characterized therein that the outer surfaces of said photoradiators being spaced apart by a minimum distance $(d_1, d_2, d_3)$ which is greater than zero and not more than about 2 mm, in a way that the contact area per unit culture medium $(S/W_1; S'/W_2; S''/W_3;$ wherein S, S' and S'' is the area, in square metres, of the photoradiators (12, 32, 62) in contact with the culture medium and $W_1$, $W_2$ and $W_3$ is the volume of culture medium, in litres, allowed to flow through the space between the photoradiators (12, 32, 62)) is greater than or equal to 1.

2. An apparatus as claimed in Claim 1, in which the reaction bath (10, 30, 60) has a hermetically sealed structure.

3. An apparatus as claimed in Claim 1, in which each of the photoradiators (12, 32, 62) radiate light therefrom in an even intensity distribution.

4. An apparatus as claimed in Claim 1, further comprising optical fibers (14) optically connected with the photoradiators to guide light into the photoradiators, respectively.

5. An apparatus as claimed in Claim 4, in which the light guided by the optical fibers is at least one of natural light and artificial light.

6. An apparatus as claimed in Claim 4, further comprising a fluorescent lamp optically connected with the photoradiators (12, 32, 62) to output light into the photoradiators.

7. An apparatus as claimed in Claim 6, in which a predetermined number of the photoradiators (12, 32, 62) radiate the output light of the optical fibers and the rest radiates the output light of the fluorescent lamp.

8. An apparatus as claimed in Claim 6, in which each of the photoradiators (12, 32, 62) associated with the optical fiber has a diameter which is smaller than the diameter of each of the photoradiators associated with the fluorescent lamp.

9. An apparatus as claimed in Claim 1, in which each of the photoradiators has a hollow cylindrical shape the diameter of which is about 10 mm, the photoradiators (12) being spaced about 1—1.2 mm from each other.

10. An apparatus as claimed in Claim 1, in which each of the photoradiators (32) has an equilateral triangular cross-section.

11. An apparatus as claimed in Claim 10, in which the reaction bath (10, 30, 60) has a cross-section of at least one of hexagon, circle, triangle and square.

12. An apparatus as claimed in Claim 1, in which each of the photoradiators (62) has a square cross-section.

13. An apparatus as claimed in Claim 12, in which the reaction bath has a cross-section of at least one of hexagon and circle.

14. An apparatus as claimed in Claim 1, in which each of the photoradiators (12) has a hexagonal cross-section.

15. An apparatus as claimed in Claim 14, in which the reaction bath (90) has a cross-section of at least one of hexagon and circle.

**Patentansprüche**

1. Photosynthesegerät mit einem Photosynthesereaktionsbad (10, 30, 60) und einer Mehrzahl von Photoradiatoren (12, 32, 62), die in dem Reaktionsbad angeordnet sind und die Form einer schmalen Röhre haben, durch gekennzeichnet, daß die Außenflächen der Photoradiatoren in einem Minimalabstand (d1, d2, d3) zueinander angeordnet sind, der größer als Null ist und nicht mehr als Ca. 2 mm beträgt, derart, daß die Kontaktfläche pro Einheit des Züchtmediums $(S/W_1; S'/W_2; S''/W_3;$ wobei S, S' und S'' der Bereich ist der Photoradiatoren (12, 32, 62) in Kontakt mit dem Pflanzmedium in Quadratmetern und $W_1$, $W_2$ und $W_3$ das Volumen des Pflanzmediums in Litern ist, das durch den Raum zwischen den Photoradiatoren (12, 32, 62) fließt größer oder gleich 1 ist.

2. Vorrichtung nach Anspruch 1, wobei das Reaktionsbad (10, 30, 62) hermetisch abgeschlossen ist.

8

3. Vorrichtung nach Anspruch 1, wobei jeder Photoradiator (12, 32, 62) das Licht mit einer gleichförmigen Intensitätsverteilung abstrahlt.

4. Vorrichtung nach Anspruch 1, wobei weiterhin optische Fasern (14) vorgesehen sind, die optisch mit den Photoradiatoren verbunden sind, um Licht in die entsprechenden Photoradiatoren einzukoppeln.

5. Vorrichtung nach Anspruch 4, wobei das von den optischen Fasern eingekoppelte Licht wenigstens Kunstlicht oder natürliches Licht ist.

6. Vorrichtung nach Anspruch 4, die weiterhin eine Fluoreszenzlampe umfaßt, die optische mit den Photoradiatoren (12, 32, 62) verbunden ist, um Licht in die Photoradiatoren einzukoppeln.

7. Vorrichtung nach Anspruch 6, wobei eine bestimmte Anzahl von Photoradiatoren (12, 32, 62) das von den optischen Fasern eingekoppelte Licht abstrahlt und die übrigen Licht von der Fluoreszenzlampe abstrahlen.

8. Vorrichtung nach Anspruch 6, wobei jeder Photoradiator (12, 32, 62), der mit einer optischen Faser verbunden ist, einen Durchmesser hat, der kleiner als der Durchmesser jedes Photoradiators ist, der mit der Fluoreszenzlampe verbunden ist.

9. Vorrichtung nach Anspruch 1, wobei jeder Photoradiator die Form eines Hohlzylinders hat, dessen Durchmesser ca. 10 mm beträgt, und wobei die Photoradiatoren (12) mit einem Abstand von 1 bis 1,2 mm zueinander angeordnet sind.

10. Vorrichtung nach Anspruch 1, wobei jeder Photoradiator (32) die Querzschnittform eines gleichseitigen Dreiecks hat.

11. Vorrichtung nach Anspruch 10, wobei das Reaktionsbad (10, 30, 60) im Querschnitt wenigstens sechseckig, kreisförmig, dreieckig oder quadratisch ist.

12. Vorrichtung nach Anspruch 1, wobei jeder Photoradiator (62) einen quadratischen Querschnitt hat.

13. Vorrichtung nach Anspruch 12, wobei das Reaktionsbad im Querschnitt wenigstens sechseckig oder kreisförmig ist.

14. Vorrichtung nach Anspruch 1, wobei jeder Photoradiator (12) im Querschnitt sechseckig ist.

15. Vorrichtung nach Anspruch 14, wobei das Reaktionsbad im Querschnitt wenigstens sechseckig oder kreisförmig ist.

## Revendications

1. Un appareil de photosynthèse comprenant un bain de réaction pour photosynthèse (10, 30, 60) et une pluralité de photoradiateurs (12, 32, 62) disposés dans ledit bain de réaction, ayant une configuration tubulaire étroite, caractérisé en ce que les surfaces extérieures desdits photoradiateurs sont espacées l'une de l'autre d'und distance minimal ($d_1$, $d_2$, $d_3$) qui est supérieure à zéro et non supérieure à environ 2 mm, d'une manière telle que la surface de contact par unité volumique du milieu de culture ($S/W_1$; $S'/W_2$; $S''/W_3$, où S, S' et S'' désignent la surface, en mètres carrés, des photoradiateurs (12, 32, 62) en contact avec le milieu de culture et $W_1$, $W_2$ et $W_3$ désignent le volume du milieu de culture, en litres, pouvant s'écouler dans l'espace existant entre les photoradiateurs (12, 32, 62)) est soit supérieure ou égale à 1.

2. Un appareil tel que revendiqué dans la revendication 1, dans lequel le bain de réaction (10, 30, 60) a une structure hermétiquement scellée.

3. Un appareil tel que revendiqué dans la revendication 1, dans lequel chacun des photoradiateurs (12, 32, 62) rayonne de la lumière avec une distribution d'intensité uniforme.

4. Un appareil tel que revendiqué dans la revendication 1, comprenant en outre des fibres optiques (14) reliées optiquement avec les photoradiateurs de façon à guider de la lumière dans les photoradiateurs respectifs.

5. Un appareil tel que revendiqué dans la revendication 4, dans lequel la lumière guidée par les fibres optiques est au moins une des lumières constituées par la lumière naturelle et la lumière artificielle.

6. Un appareil tel que revendiqué dans la revendication 4, comprenant en outre une lampe fluorescente reliée optiquement avec les photoradiateurs (12, 32, 62) pour fournir de la lumière aux photoradiateurs.

7. Un appareil tel que revendique dans la revendication 6, dans lequel un nombre prédéterminé de photoradiateurs (12, 32, 62) rayonnent la lumière de sortie des fibres optiques et le reste des photoradiateurs rayonne la lumière de sortie de la lampe fluorescente.

8. Un appareil tel que revendiqué dans la revendication 6, dans lequel chacun des photoradiateurs (12, 32, 62) associés à la fibre optique a un diamètre qui est plus petit que le diamètre de chacun des photoradiateurs associés à lampe fluorescente.

9. Un appareil tel que revendiqué dans la revendication 1, dans lequel chacun des photoradiateurs a une forme cylindrique creuse dont le diamètre est environ de 10 mm, les photoradiateurs (12) étant espacés l'un de l'autre d'environ 1 à 1,2 mm.

10. Un appareil tel que revendiqué dans la revendication 1, dans lequel chacun des photoradiateurs (32) a une section droite en forme de triangle équilatéral.

11. Un appareil tel que revendique dans la revendication 10, dans lequel le bain de réaction (10, 30, 60) a une section droite ayant au moins la forme d'un hexagone, d'un cercle, d'un triangle ou d'un carré.

12. Un appareil tel que revendiqué dans la revendication 1, dans lequel chacun des photoradiateurs (62) a une section droite de forme carrée.

**0 081 156**

13. Un appareil tel que revendiqué dans la revendication 12, dans lequel le bain de réaction a une section droite ayant au moins une forme d'hexagone et de cercle.

14. Un appareil tel que revendiqué dans la revendication 1, dans lequel chacun des photoradiateurs (12) a une section droite hexagonale.

15. Un appareil tel que revendiqué dans la revendication 14, dans lequel le bain de réaction (90) a une section droite ayant au moins la forme d'un hexagone et d'un cercle.

# Fig. 1

# Fig. 2

## Fig. 3

## Fig. 4

## Fig. 5

2

## Fig. 6

## Fig. 7

## Fig. 8

## Fig. 9

## Fig. 10

## Fig. 11